# NEUE EUROPÄISCHE PATENTSCHRIFT

(11) **EP 1 204 382 B2**
(45) Veröffentlichungstag und Bekanntmachung des Hinweises auf die Entscheidung über den Einspruch: **27.09.2006**
(45) Hinweis auf die Patenterteilung: 25.06.2003
(21) Anmeldenummer: 99941622.5
(22) Anmeldetag: 14.08.1999
(51) Int. Cl.: A61B 17/86

(54) **KNOCHENSCHRAUBE**
BONE SCREW
VIS POUR OS

(43) Veröffentlichungstag der Anmeldung: 15.05.2002
(73) Patentinhaber: Aesculap AG & Co. KG, 78532 Tuttlingen (DE)
(72) Erfinder: SCHULTHEISS, Markus, D-89278 Nersingen (DE); CLAES, Lutz, D-89233 Neu-Ulm (DE); KINZL, Lothar, D-89081 Ulm (DE)
(74) Vertreter: Boehme, Ulrich
(86) Internationale Anmeldenummer: PCT/EP1999/005973
(87) Internationale Veröffentlichungsnummer: WO 2001/012088

(56) Entgegenhaltungen:
- US-A- 4 537 185
- US-A- 4 653 489
- PATENT ABSTRACTS OF JAPAN vol. 1997, no. 10, 31. Oktober 1997 (1997-10-31) & JP 09 149906 A (NAGOYA RASHI SEISAKUSHO:KK), 10. Juni 1997 (1997-06-10)

## Beschreibung

Die Erfindung betrifft eine Knochenschraube mit einem Schraubenkörper, welcher mit einem Außengewinde versehen ist und welcher Durchflußmittel für Knochenzement zur Herstellung eines Zementmantels um die Knochenschraube aufweist.

Derartige Knochenschrauben werden beispielsweise als Verankerungsschrauben für ein Stabilisierungssystem eingesetzt, wenn mehrere Knochenelemente miteinander zu verbinden sind. Die Herstellung eines Zementmantels um die Knochenschraube nach der Implantierung erhöht die Verankerungsstabilität. Dies ist besonders wichtig, wenn die Knochenqualität reduziert ist, wie z.B. bei osteoporotischem Knochengewebe.

Aus der EP 0 305 417 B1 ist eine Knochenschraube bekannt, welche einen longitudinalen Kanal aufweist entlang dem eine Mehrzahl von sich radial erstreckenden transversalen Kanälen angeordnet sind, die in Kontakt stehen mit diesem longitudinalen Kanal. Eine weitere Knochenschraube ist aus der US 4,653,489 bekannt, die mit einer Anzahl von Querbohrungen versehen ist, welche sich jeweils zwischen zwei Gewindegängen erstrecken.

In dem Zeitschriftenartikel L. Claes et al. "Die Verbesserung der Primärstabilität von DHS-Osteosynthesen ..." in *Unfallchirurg (1995) 98:118-123* ist eine dynamische Hüftschraube beschrieben, in die im Gewindeteil eine parallel zur Schraubenachse verlaufende Nut eingefräst ist, dadurch soll die Verteilung eines Knochenzementes gefördert werden.

Aus der WO 97/15246 ist ein Zwischenwirbelimplantat bekannt, bei dem ein in einem Käfig drehbar gelagerter Rotationskörper mit einem Außengewinde versehen ist, so daß durch Drehung des Rotationskörpers das gesamte Implantat in den Zwischenwirbelraum eingezogen werden kann. Dieser Rotationskörper weist einen Innenraum auf, der über Durchbrechungen mit dem Außenraum in Verbindung steht. Der Innenraum soll mit Knochenspänen und Knochenersatzstoffen ausgefüllt werden, die durch die Durchbrechungen unter Ausbildung einer knöchernen Verbindung zu den benachbarten Knochen hindurchwachsen sollen.

Ausgehend von dem eingangs beschriebenen Stand der Technik liegt der Erfindung die Aufgabe zugrunde, eine Knochenschraube zu schaffen, die gegenüber aus dem Stand der Technik bekannten Knochenschrauben eine erhöhte Verankerungsstabilität aufweist und dies insbesondere auch bei der Implantierung in Knochen mit reduzierter Qualität.

Diese Aufgabe wird bei einer Knochenschraube der eingangs beschriebenen Art erfindungsgemäß dadurch gelöst, daß die Knochenschraube in Längsrichtung des Schraubenkörpers aufeinanderfolgende Abschnitte aufweist, welche sich in der Ausbildung einer Außenfläche unterscheiden, daß die Durchflußmittel mindestens eine Längsausnehmung umfassen, welche quer zu einer radialen Richtung und quer zu einer Umfangsrichtung im Schraubenkörper gebildet ist, daß die mindestens eine Längsausnehmung in ihrer Längsrichtung so dimensioniert ist, daß sie sich über mehrere Windungen des Außengewindes erstreckt, daß ein mittlerer Abschnitt des Schraubenkörpers im wesentlichen zylindrisch ausgebildet ist, daß ein hinterer Abschnitt des Schraubenkörpers im wesentlichen konisch oder kegelstumpfförmig ausgebildet ist und daß die mindestens eine Längsausnehmung im wesentlichen im mittleren Abschnitt angeordnet ist.

Durch eine derartige Ausbildung und Anordnung einer Ausnehmung, mittels welcher Knochenzement in den Zwischenraum zwischen einer implantierten Knochenschraube und einem Knochenlager bringbar ist, läßt sich ein gleichmäßiger Zementmantel um die Schraube ausbilden. Dies bewirkt eine hohe Verankerungsstabilität der erfindungsgemäßen Knochenschraube. Mittels einer Längsausnehmung, die im wesentlichen längs des Schraubenkörpers gebildet ist, lassen sich alle Bereiche zwischen den Windungen des Außengewindes mit Knochenzement versorgen, wobei ein guter Strömungsfluß für Knochenzement erreichbar ist. Bei der Knochenschraube der EP 0 305 417 B1 dahingegen muß im wesentlichen für jeden Zwischenraum ein eigener Radialkanal vorgesehen werden. Außerdem muß, damit sich auch im Bereich um ein vorderes Schraubenende ein Zementmantel ausbilden kann, der dortige Längskanal durch den ganzen Schraubenkörper durchgehen. Dadurch ist die Ausbildung als selbstschneidende Knochenschraube beeinträchtigt. Bei der erfindungsgemäßen Knochenschraube dahingegen genügt grundsätzlich eine Längsausnehmung und durch entsprechende Ausnehmungen in den Windungen des Außengewindes läßt sich auch ein Zementmantel um das vordere Ende der Knochenschraube bilden. Durch die Erstreckung der Längsausnehmung über mehrere Windungen des Außengewindes läßt sich im wesentlichen mit einer Längsausnehmung der Knochenzement für den ganzen Schraubenkörper zuführen.

Durch die unterschiedliche Ausbildung der Außenflächen in ihrer geometrischen Gestalt läßt sich eine besonders hohe Verankerungsstabilität erreichen, indem einerseits ein oder mehrere Abschnitte ausgebildet werden, die für einen guten Gewindehalt im Knochen sorgen und andererseits Abschnitte ausgebildet werden, die eine Dichtungswirkung gegenüber dem Austreten von Knochenzement aufweisen. Auf diese Weise läßt sich ein Zementmantel ausbilden, der durch eine formschlüssige Verzahnung mit dem Knochen die Verankerungskräfte in allen Richtungen erhöht. Mittels geeigneter Wahl der Abmessungen der Abschnitte läßt sich auch das Knochenzement-Volumen, das zugeführt werden muß, genau einstellen. Dadurch lassen sich die mit einer Erwärmung durch Zementreaktionen verbundenen Probleme weitgehend vermeiden. Da der mittlere Abschnitt des Schraubenkörpers im wesentlichen zylindrisch ausgebildet ist, ergibt sich ein guter Gewindehalt einer implantierten Schraube in dem Knochen. Außerdem läßt sich dadurch eine gleichmäßige Dicke des sich ausbildenden Zementmantels erreichen. Die konische oder kegelstumpfförmige Ausgestaltung des hinteren Abschnittes führt zu einer Dichtwirkung gegenüber dem unbeabsichtigten Austreten von Knochenzement aus einem Knochenlager bei implantierter Knochenschraube.

Besonders vorteilhaft ist es, wenn die Durchflußmittel einen im Schraubenkörper gebildeten Hohlkanal umfassen und wenn eine Längsausnehmung mit dem Hohlkanal verbunden ist. Dadurch kann in den Hohlkanal eingeführter Knochenzement direkt und ohne wesentliche Strömungsbarrieren über eine solche Längsausnehmung aus dem Schraubenkörper hinaustreten, um so einen gleichmäßigen Zementmantel um die Knochenschraube auszubilden. Dadurch läßt sich gewährleisten, daß die erfindungsgemäße Knochenschraube nach der Implantierung eine hohe Verankerungsstabilität aufweist. Günstigerweise ist eine Längsausnehmung in ihrer Längsrichtung mit dem Hohlkanal verbunden, um den Strömungswiderstand für den Knochenzement zu minimieren.

Bei einer besonders vorteilhaften Ausführungsform sind einer Längsausnehmung entsprechende Ausnehmungen in Windungen des Außengewindes zugeordnet. Dadurch wird der Fluß von Knochenzement zwischen verschiedenen Zwischenbereichen, d.h. den Bereichen zwischen benachbarten Gewindewindungen, ermöglicht, um so eine gleichmäßige Verteilung des Knochenzementes und damit eine gleichmäßige Ausbildung des Zementmantels um eine implantierte Knochenschraube zu ermöglichen. Günstigerweise ist eine Windung des Außengewindes im Bereich einer Längsausnehmung durchbrochen, um so den Durchfluß für Knochenzement zu ermöglichen.

Fertigungstechnisch besonders günstig ist es, wenn eine Längsausnehmung schlitzförmig ausgebildet ist. Dadurch läßt sich diese durch spanabhebende Materialbearbeitung, wie beispielsweise Fräsbearbeitung, auf einfache Weise herstellen. Vorteilhafterweise ist eine Schlitzebene im wesentlichen senkrecht zu einer Umfangsrichtung des Schraubenkörpers. Der Schlitz ist dabei insbesondere durch eine obere Schlitzebene und eine untere Schlitzebene gebildet, die parallel zueinander sind. Durch die Ausbildung senkrecht zu einer Umfangsrichtung läßt sich die Schlitzebene in radialer Richtung orientieren. Dies ermöglicht bei einem rotationssymmetrischen Schraubenkörper einen guten Durchfluß von Knochenzement. Weiterhin ist es dann günstig, wenn eine Schlitzebene im wesentlichen in einer radialen Richtung bezüglich des Schraubenkörpers liegt.

Besonders vorteilhaft ist es, wenn eine Schlitzebene im wesentlichen in Längsrichtung bezüglich des Schraubenkörpers liegt. Dies gewährleistet zum einen eine einfache Herstellbarkeit einer solchen Längsausnehmung und zum anderen wird ein guter Durchfluß für Knochenzement erreicht.

Zur Ausbildung eines eine hohe Verankerungsstabilität gewährleistenden Zementmantels ist es besonders vorteilhaft, wenn eine Mehrzahl von Längsausnehmungen über den Umfang des Schraubenkörpers angeordnet sind. Dadurch lassen ich die Bereiche zwischen den Windungen des Außengewindes bezogen auf den Umfang des Schraubenkörpers gleichmäßig mit Knochenzement versorgen.

Bei einer vorteilhaften Variante einer Ausführungsform sind drei Längsausnehmungen über den Umfang des Schraubenkörpers angeordnet. Dadurch ist zum einen die Anzahl der Durchbrechungen in den Windungen des Außengewindes und im Schraubenkörper gering gehalten und andererseits läßt sich eine gleichmäßige Knochenzementbeaufschlagung erreichen.

Ganz besonders vorteilhaft dabei ist es, wenn die Mehrzahl von Längsausnehmungen im wesentlichen symmetrisch bezüglich einer Längsachse des Schraubenkörpers angeordnet sind. Auf diese Weise strömt Knochenzement, der in den Schraubenkörper eingeführt wird, über die Längsausnehmungen gleichmäßig in die Bereiche zwischen den Windungen des Außengewindes und bildet einen gleichmäßigen Zementmantel aus.

Günstigerweise ist ein vorderer Abschnitt des Schraubenkörpers im wesentlichen konisch oder kegelstumpfförmig ausgebildet. Dadurch läßt ich eine gute Gewindeschneidefunktion erreichen: Andererseits wirkt ein solcher Abschnitt dichtend gegenüber dem unbeabsichtigten Austreten von Knochenzement.

Günstigerweise ist dabei eine Hüllfläche von Windungen des Außengewindes im vorderen Abschnitt im wesentlichen konisch oder kegelstumpfförmig, um eine gute Schneidefunktion für das Gewinde zu erreichen. Günstigerweise ist eine Hüllfläche des Schraubenkörpers im vorderen Abschnitt im wesentlichen konisch oder kegelstumpfförmig, um eine gute Dichtwirkung gegenüber dem unbeabsichtigten Austreten von Knochenzement zu erreichen.

Günstigerweise ist eine Hüllfläche von Windungen des Außengewindes im mittleren Abschnitt im wesentlichen zylindrisch und ist ebenfalls die Hüllfläche des Schraubenkörper im mittleren Abschnitt, im wesentlichen zylindrisch.

Ganz besonders vorteilhaft ist es, wenn ein-Hohlkanal im Schraubenkörper durch den mittleren Abschnitt geführt ist und insbesondere bis oder in die Nähe des vorderen Abschnitts geführt ist. Der Hohlkanal braucht dann nicht durchgehend ausgebildet zu sein, so daß die Schneidwirkung am vorderen Ende des Gewindes nicht beeinträchtigt wird und anderseits läßt sich die Knochenschraube trotzdem gleichmäßig mit Knochenzement beaufschlagen, um einen Zementmantel auszubilden. Aus den gleichen Gründen ist es vorteilhaft, wenn die mindestens eine Längsausnehmung im wesentlichen im mittleren Abschnitt angeordnet ist.

Ganz besonders günstig dabei ist es, wenn eine Hüllfläche des Schraubenkörpers im hinteren Abschnitt im wesentlichen konisch oder kegelstumpfförmig ausgebildet ist, um die genannte Dichtwirkung zu erreichen, und eine Hüllfläche von Windungen des Außengewindes im mittleren Abschnitt im wesentlichen zylindrisch ist. Dadurch läßt sich zum einen die Dichtwirkung erreichen und zum anderen ist die Beeinflussung des Knochens durch die Schraubenbohrung dadurch nicht vergrößert. Günstigerweise fällt dabei eine Hüllfläche von Windungen im hinteren Abschnitt und von Windungen im mittleren Abschnitt zusammen.

Fertigungstechnisch vorteilhaft ist es, wenn ein Konuswinkel des vorderen Abschnittes im wesentlichen einem Konuswinkel des hinteren Abschnittes entspricht.

Um einen gleichmäßigen Zementmantel um die Knochenschraube auszubilden und insbesondere die erfindungsgemäße Knochenschraube auch in beispielsweise osteoporotischem Gewebe mit hoher Stabilität zu verankern, ist es vorgesehen, daß die Windungen des Außengewindes in einem Bereich, in dem keine Längsausnehmungen angeordnet sind, mit Längsdurchbrüchen versehen sind. Dadurch kann im wesentlichen der gesamte Bereich um den Schraubenkörper mit Knochenzement versorgt werden, indem Knochenzement aus denjenigen Zwischenräumen, die über Längsausnehmungen versorgt werden, durch die Längsdurchbrechungen auch in nicht direkt versorgbare Bereiche fließen kann. So läßt sich beispielsweise auch um den vorderen Abschnitt der erfindungsgemäßen Knochenschraube ein Zementmantel ausbilden.

Ganz besonders günstig und fertigungstechnisch besonders einfach ist es, wenn eine Oberfläche des Schraubenkörpers zwischen benachbarten Windungen des Außengewindes im wesentlichen parallel zur Längsrichtung des Schraubenkörpers ist. Dadurch wird dem Knochenzement ein größerer Zwischenraum bereitgestellt und insbesondere läßt sich in den Bereichen zwischen den einzelnen Windungen des Außengewindes für Verteilung um den Umfang des Schraubenkörpers eine gute Strömungsführung für den Knochenzement erreichen.

Fertigungstechnisch besonders einfach ist es, wenn der Hohlkanal durch eine Sacklochbohrung gebildet ist.

Vielfältige Einsatzmöglichkeiten ergeben sich, wenn der Schraubenkörper mit einem Innengewinde versehen ist. Dadurch läßt sich insbesondere ein Schraubenkopf einsetzen, der für eine entsprechende Anwendung angepaßt sein kann, beispielsweise ein Kugelkopf für einen polyaxialen Spannmechanismus. Günstigerweise ist dabei ein solcher Schraubenkopf mit einem Kanal versehen, der mit dem im Schraubenkörper gebildeten Hohlkanal verbindbar ist, um auf diese Weise Knochenzement dem Hohlkanal zuführen zu können.

Ganz besonders vorteilhaft ist es, wenn der Schraubenkörper ein Ankopplungselement für einen Knochenzementapplikator aufweist. Ein solches Ankopplungselement kann beispielsweise in einem Schraubenkopf, welcher einstückig mit dem Schraubenkörper gebildet oder mit diesem verbunden ist, angeordnet sein oder direkt in dem Schraubenkörper angeordnet sein. Vorteilhafterweise umfaßt das Ankopplungselement eine Aufnahme für eine Düse eines Knochenzementapplikators, um so auf einfache Art und Weise Knochenzement in den Schraubenkörper einspritzen zu können.

Eine besonders gute Verankerungsstabilität ergibt sich, wenn ein Durchmesser des Schraubenkörpers größer ist als eine Ganghöhe des Außengewindes. Weiterhin läßt sich eine hohe Verankerungsstabilität erreichen, wenn eine erfindungsgemäße Knochenschraube in etwa sieben bis zwölf Windungsumläufe des Außengewindes umfaßt.

Vorteilhafterweise ist das Außengewinde so ausgebildet, daß die Knochenschraube selbstschneidend ist, um sie auf diese Weise universell einsetzbar zu machen.

Die nachfolgende Beschreibung bevorzugter Ausführungsformen der Erfindung dient im Zusammenhang mit der Zeichnung der näheren Erläuterung.

Es zeigen:
- Fig. 1: Eine perspektivische Ansicht eines Ausführungsbeispiels einer erfindungsgemäßen Knochenschraube;
- Fig. 2: eine seitliche Schnittansicht der Knochenschraube gemäß Fig. 1;
- Fig. 3: eine Schnittansicht entlang der Linie A-A der Fig. 2;
- Fig. 4: eine Seitenansicht einer weiteren Ausführungsform einer erfindungsgemäßen Knochenschraube;
- Fig. 5: eine Seitenansicht der Ausführungsform gemäß Fig. 1 mit einem eingesetzten Schraubenkopf und
- Fig. 6: eine schematische Darstellung eines Knochenzementapplikators, welcher an die Schraube gemäß Fig. 5 angesetzt ist, um Knochenzement zu applizieren.

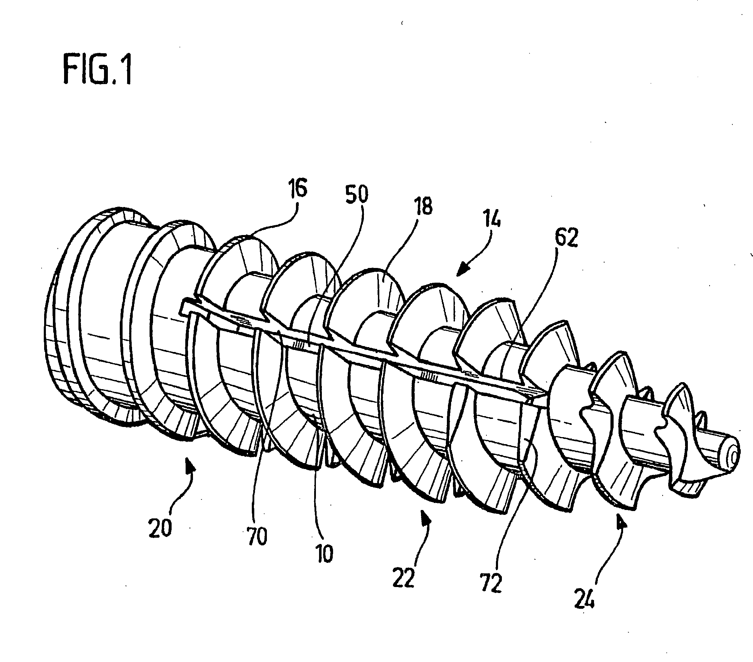

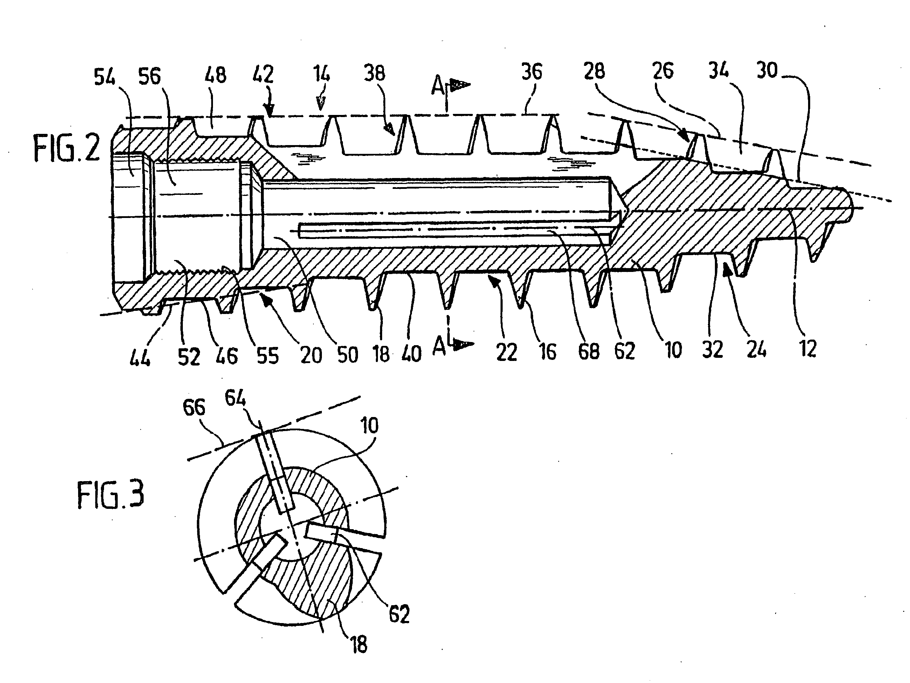

Eine erfindungsgemäße Knochenschraube umfaßt, wie in den Fig. 1 und 2 gezeigt, einen Schraubenkörper 10, der im wesentlichen rotationssymmetrisch zu einer Längsachse 12 ist. Der Schraubenkörper ist mit einem als Ganzes mit 14 bezeichneten Außengewinde versehen. Dieses Gewinde 14 umfaßt eine Mehrzahl von Windungen 16 mit Windungsflanken 18, welche insbesondere so angeordnet und ausgebildet sind, daß die erfindungsgemäße Knochenschraube selbstschneidend ist. Das Außengewinde 14 isteinstükkig am Schraubenkörper 10 gebildet. Ein bevorzugtes Material für die Knochenschraube ist beispielsweise Titan.

Die erfindungsgemäße Knochenschraube weist in Längsrichtung des Schraubenkörpers 10 aufeinanderfolgende Abschnitte 20,22,24 auf, welche sich in der Ausbildung ihrer äußeren Gestalt unterscheiden. Ein vorderer Abschnitt24, welcher das distale Ende der Knochenschraube bildet, ist dabei kegelförmig ausgebildet. Eine Hüllfläche 26 der Windungen 28 des Außengewindes 14 in diesem vorderen Abschnitt 24 ist konisch mit einem Konuswinkel, der beispielsweise im Bereich zwischen 5° und 15° und insbesondere bei ca. 10° liegt. Diese Hüllfläche 26 ist rotationssymmetrisch zur Längsachse 12 des Schraubenkörpers 10. Des weiteren weist der Schraubenkörper 10 im vorderen Abschnitt 24 eine Hüllfläche 30 auf, die ebenfalls konisch ist und koaxial zur Hüllfläche 26, d.h. im wesentlichen den gleichen Konuswinkel aufweist wie die Hüllfläche 26. Die Hüllfläche 30 erhält man durch den Schnitt zwischen den Windungsflanken 18 der Windungen 28 und dem Schraubenkörper 10 im vorderen Abschnitt 24, wie in Fig. 2 gezeigt. Eine äußere Oberfläche 32 des Schraubenkörpers 10 im vorderen Abschnitt 24 selber, welche zwischen den Windungen 28 gebildet ist, ist zylindrisch, d.h. parallel zur Längsachse 12 und folglich nicht zu dieser geneigt, um dadurch einen größeren Zwischenraum 34 zwischen benachbarten Windungen 28 bereitzustellen.

An den vorderen Abschnitt 24 schließt sich in proximaler Richtung ein mittlerer Abschnitt 22 mit einer Hüllfläche 36 an, welche zylindrisch ist; die Windungen 38 im mittleren Abschnitt 22 weisen daher die gleiche Höhe auf. Eine äußere Oberfläche 40 des Schraubenkörpers 10 im mittleren Bereich 22 ist ebenfalls zylindrisch und koaxial zur Hüllfläche 36.

An den mittleren Abschnitt 22 schließt sich in proximaler Richtung ein hinterer Abschnitt 20 an, dessen Windungen 42 die gleiche Hüllfläche wie die Windungen 38 im mittleren Abschnitt 22 aufweisen, d.h. die Windungen 42 haben eine zylindrische Hüllfläche. Eine Hüllfläche 44 des Schraubenkörpers 10, welche durch den Schnitt der Windungen 42 mit dem Schraubenkörper 10 im hinteren Abschnitt 20 definiert ist, ist kegelförmig mit einem Kegelwinkel, der beispielsweise bei ca. 10° liegt. Eine äußere Oberfläche 46 des Schraubenkörpers 10 im hinteren Abschnitt 20 zwischen benachbarten Windungen 42 ist dabei zylindrisch ausgebildet, um ähnlich wie beim vorderen Abschnitt 24 größere Zwischenräume 48 für Knochenzement zwischen benachbarten Windungen 42 im hinteren Abschnitt 20 bereitzustellen. Die Windungen 42 über dem Schraubenkörper 10 weisen daher im hinteren Abschnitt 20 unterschiedliche Höhen gegenüber dem Schraubenkörper 10 auf.

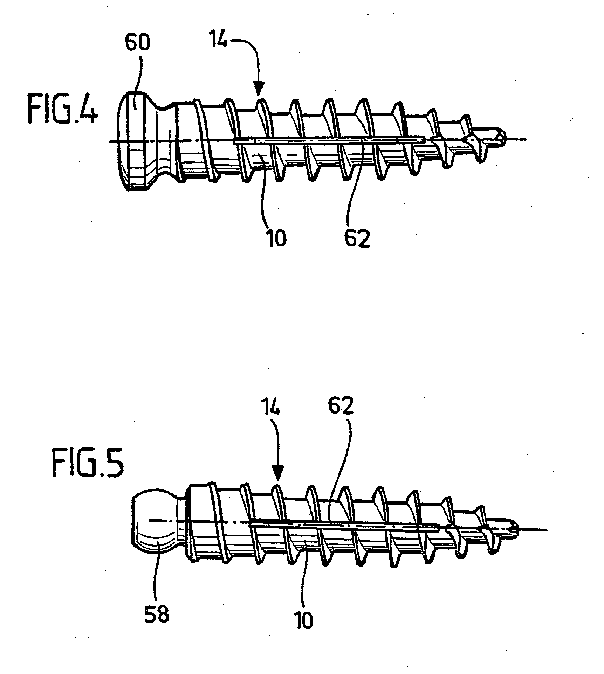

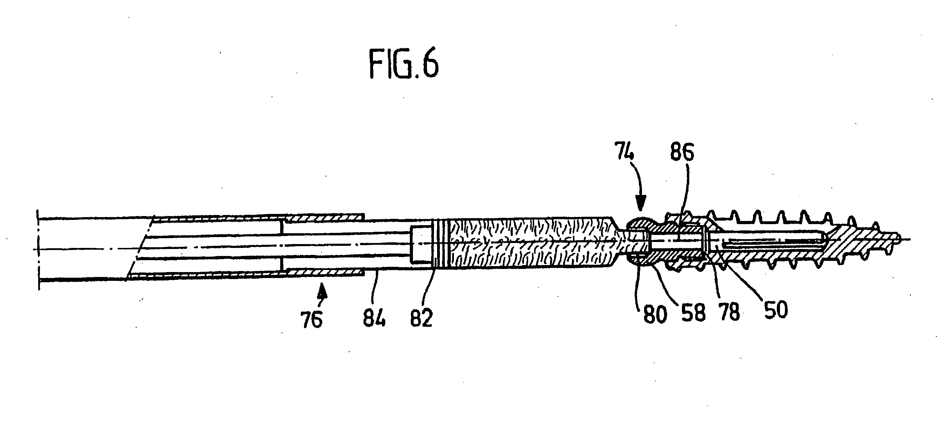

Die Knochenschraube umfaßt Durchflußmittel, mittels denen Knochenzement durch den Schraubenkörper 10 hindurch in dieZwischenräume 34, 48 und besonders zwischen die Windungen 38 im mittleren Abschnitt 22 eingebracht werden kann. Dazu ist in dem Schraubenkörper 10 koaxial zur Längsachse 12 ein Hohlkanal 50 gebildet, beispielsweise durch eine Sacklochbohrung, der sich durch den hinteren Abschnitt 20 und den mittleren Abschnitt 22 bis in den Übergangsbereich zwischen dem mittleren Abschnitt 22 und dem vorderen Abschnitt 24 erstreckt. Bei dem in Fig. 2 gezeigten Ausführungsbeispiel weist der Schraubenkörper 10 im hinteren Abschnitt 20 eine Ausnehmung 52 auf, an die der Hohlkanal 50 anschließt. Die Ausnehmung 52 umfaßt einen ersten zylindrischen Bereich 54 und einen zweiten zylindrischen Bereich 56, wobei der erste zylindrische Bereich 54 einen größeren Durchmesser aufweist als der zweite zylindrische Bereich 56. Die Ausnehmung 52 ist mit einem Innengewinde 55 versehen. In diese Ausnehmung 52 kann, wie in Fig. 5 und 6 gezeigt, ein Schraubenkopf 58 in das Innengewinde 55 eingesetzt sein, der je nach Anwendungsfall entsprechend ausgebildet ist. Es ist auch möglich, einen Schraubenkopf 60 einstückig mit der Knochenschraube auszubilden, wie in Fig. 4 gezeigt.

Im Schraubenkörper 10 ist als Durchflußmittel für Knochenzement mindestens eine Längsausnehmung 62 gebildet, die quer und insbesondere senkrecht zu einer radialen Richtung 64 und quer und insbesondere senkrecht zu einer Umfangsrichtung 66 angeordnet ist (Fig. 3). Durch solche Längsausnehmungen 62 kann Knochenzement über den Hohlkanal 50 in Zwischenräume zwischen den Windungen 16 fließen. Eine Längsausnehmung 62 weist eine Längsachse 68 auf, welche bevorzugterweise parallel zur Längsachse 12 des Schraubenkörpers 10 ist. Die Abmessungen der Längsausnehmung 62 sind so gewählt, daß eine Länge der Längsausnehmung 62 in Richtung ihrer Längsachse 68 wesentlich größer ist als die Breite quer dazu und sich eine Längsausnehmung 62 über eine Mehrzahl von Windungen 16 im mittleren Abschnitt 22 erstreckt. In dem in Fig. 2 gezeigten Ausführungsbeispiel erstreckt sich eine Längsausnehmung über die Windungen 38 im mittleren Abschnitt 22 bis in den hinteren Abschnitt 20.

Eine Längsausnehmung 62 ist schlitzförmig ausgebildet (Fig. 3), wobei eine Schlitzebene in Längsrichtung 12 und radialer Richtung 64 liegt. Die Schlitzebene erstreckt sich auch über die Windungen 16, d.h. in den Windungen 16 sind bei den Längsausnehmungen 62 und diesen zugeordnet durchbrochene Ausnehmungen 70 (Fig. 1) gebildet, durch die die Zwischenräume zwischen benachbarten Windungen 16 miteinander korrespondieren. Die Windungen 28 im vorderen Abschnitt 24 der Knochenschraube und insbesondere solche Windungen, über die sich eine Längsausnehmung 62 nicht mehr erstreckt, weisen größere Durchbrechungen 72 auf. Auf diese Weise kann Knochenzement in Zwischenräume 34 fließen, welche selber nicht direkt mit dem Hohlkanal 50 verbunden sind (sondern nur noch indirekt über die Durchbrechungen 72).

Eine Knochenschraube umfaßt, wie in Fig. 3 gezeigt, bevorzugterweise eine Mehrzahl von Längsausnehmungen 62 - beispielsweise drei solcher Längsausnehmungen -, die um den Umfang des Schraubenkörpers 10 angeordnet sind. Insbesondere sind die Längsausnehmungen 62 symmetrisch um die Längsachse 12 angeordnet, um die Zwischenräume zwischen den Windungen gleichmäßig mit Knochenzement beaufschlagen zu können.

Bei einer Variante einer Ausführungsform,welche in Fig. 6 gezeigt ist, ist an dem hinteren Ende des Hohlkanals 50 ein Ankopplungselement 74 für einen Knochenzementapplikator 76 angeordnet. In dem gezeigten Ausführungsbeispiel ist das Ankopplungselement 74 in dem eingesetzten Schraubenkopf 58 gebildet. Das Ankopplungselement 74 weist eine Anlagefläche 78, die insbesondere ringförmig ausgebildet ist, für eine Düse 80 des Knochenzementapplikators 76 auf.

Der Knochenzementapplikator 76 umfaßt ein Betätigungselement (in Fig. 6 nicht gezeigt) mittels dem ein Operateur die Knochenzementzuführung zu einer erfindungsgemäßen Knochenschraube steuert. Durch das Betätigungselement läßt sich ein Stempel 82 längsverschieben. In den Knochenzementapplikator 76 ist eine auswechselbare Knochenzement-Kartusche 84 eingesetzt, und durch den Stempel 82 läßt sich Knochenzement aus der Kartusche 84 durch die Düse 80 in den Hohlkanal 50 einführen. Der Schraubenkopf 58 weist dazu einen Kanal 86 auf, der mit dem Hohlkanal 50 verbunden ist.

Beispielhafte Abmessungen für eine erfindungsgemäße Knochenschraube sind eine Längenabmessung von 40 mm, ein Durchmesser des Schraubenkörpers 10 von 6 mm (im mittleren Abschnitt 22), einen Durchmesser von 10 mm des Außengewindes 14 (im hinteren Abschnitt 20 und im mittleren Abschnitt 22) und eine Ganghöhe von 4 mm des Außengewindes. Bevorzugt sind drei Längsausnehmungen 62 um den Umfang des Schraubenkörpers 10.

Die erfindungsgemäße Knochenschraube läßt sich nun wie folgt einsetzen:

Beispielsweise wird die erfindungsgemäße Knochenschraube als Verankerungsschraube eines Stabilisierungssystems bei der Verbindung von Knochenelementen verwendet. Die Knochenschraube wird durch das selbstschneidende Gewinde in einen Knochen implantiert; dies erfolgt insbesondere durch Eindrehen mittels des selbstschneidenden Außengewindes 14. Um die Verankerungsstabilitätzu erhöhen, wird zur Bildung eines Zementmantels um die Knochenschraube Knochenzement in den Zwischenraum zwischen dem Knochenlager und der in das Knochenlager eingesetzten Knochenschraube gebracht. Dazu wird beispielsweise über den Knochenzementapplikator 76 über den Schraubenkopf 58 der Knochenzement, welcher eine entsprechend niedrige Viskosität haben muß, eingespritzt und fließt über den Hohlkanal 50 durch die Längsausnehmungen 62 in die Zwischenräume zwischen den Windungen 16. In die Zwischenräume 34 in dem vorderen Abschnitt 24 der Knochenschraube kann Knochenzement über die Durchbrechungen 72 fließen. Dadurch bildet sich zwischen der Knochenschraube und dem Knochenlager ein Zementmantel, der die Knochenschraube umschließt und so sind über den Zementmantel die Verankerungskräfte der Knochenschraube durch eine formschlüssige Verzahnung mit dem Knochen in allen Richtungen erhöht. Bei reduzierter Knochenqualität, wie z.B. bei einem osteoporotischen Knochen, kann der Knochenzement auch weiter ins Knochengewebe eindringen.

Die konische Ausbildung des hinteren Abschnitts 20 und des vorderen Abschnitts 24 erhöht die Dichtwirkung der Knochenschraube gegenüber dem Knochenzementfluß, so daß ein unbeabsichtigtes Austreten von Knochenzement aus dem Knochenlager nach außen weitgehend vermieden ist.

Vergleichsversuche haben ergeben, daß die erfindungsgemäße Knochenschraube auch in osteoporotischem Knochengewebe eine gute Verankerungsstabilität aufweist. In einem humanen osteoporotischen Wirbelkörper mit einer Knochenmassendichte von ca. 240 mg/cm³ ließ sich beispielsweise eine mittlere Verankerungsstärke von ca. 550 N ohne Knochenzementanwendung und von ca. 810 N mit Knochenzementanwendung erreichen. In gesundem Gewebe ließ sich eine Verankerungsstärke erreichen, die ca. zweifach größer ist als gegenüber aus dem Stand der Technik bekannten Knochenschrauben.

## Patentansprüche

1. Knochenschraube mit einem Schraubenkörper (10), welcher mit einem Außengewinde (14) versehen ist und welcher Durchflußmittel für Knochenzement zur Herstellung eines Zementmantels um die Knochenschraube aufweist,
**dadurch gekennzeichnet, daß** die Knochenschraube in Längsrichtung (12) des Schraubenkörpers (10) aufeinanderfolgende Abschnitte (20, 22, 24) aufweist, welche sich in der Ausbildung einer Außenfläche unterscheiden, daß die Durchflußmittel mindestens eine Längsausnehmung (62) umfassen, welche quer zu einer radialen Richtung (64) und quer zu einer Umfangsrichtung (66) im Schraubenkörper (10) gebildet ist, daß die mindestens eine Längsausnehmung (62) in ihrer Längsrichtung (68) so dimensioniert ist, daß sie sich über mehrere Windungen (16) des Außengewindes (14) erstreckt, daß ein mittlerer Abschnitt (22) des Schraubenkörpers (10) im wesentlichen zylindrisch ausgebildet ist, daß ein hinterer Abschnitt (20) des Schraubenkörpers (10) im wesentlichen konisch oder kegelstumpfförmig ausgebildet ist und daß die mindestens eine Längsausnehmung (62) im wesentlichen im mittleren Abschnitt (22) angeordnet ist.

2. Knochenschraube nach Anspruch 1, **dadurch gekennzeichnet, daß** die mindestens eine Längsausnehmung (62) senkrecht zu einer radialen Richtung (64) angeordnet ist.

3. Knochenschraube nach Anspruch 1 oder 2, **dadurch gekennzeichnet, daß** die mindestens eine Längsausnehmung (62) senkrecht zu einer Umfangsrichtung (66) angeordnet ist.

4. Knochenschraube nach einem der voranstehenden Ansprüche,
**dadurch gekennzeichnet, daß** die Durchflußmittel einen im Schraubenkörper (10) gebildeten Hohlkanal (50) umfassen und daß die mindestens eine Längsausnehmung (62) mit dem Hohlkanal (50) verbunden ist.

5. Knochenschraube nach Anspruch 4, **dadurch gekennzeichnet, daß** die mindestens eine Längsausnehmung (62) in ihrer Längsrichtung (68) mit dem Hohlkanal (50) verbunden ist.

6. Knochenschraube nach einem der voranstehenden Ansprüche,
**dadurch gekennzeichnet, daß** der mindestens einen Längsausnehmung (62) entsprechende Ausnehmungen (70) in Windungen (16) des Außengewindes (14) zugeordnet sind.

7. Knochenschraube nach Anspruch 6, **dadurch gekennzeichnet, daß** eine Windung (18) des Außengewindes (14) im Bereich der mindestens einen Längsausnehmung (62) durchbrochen ist.

8. Knochenschraube nach einem der voranstehenden Ansprüche,
**dadurch gekennzeichnet, daß** die mindestens eine Längsausnehmung (62) schlitzförmig ausgebildet ist.

9. Knochenschraube nach Anspruch 8, **dadurch gekennzeichnet, daß** eine Schlitzebene im wesentlichen senkrecht zu einer Umfangsrichtung des Schraubenkörpers (10) ist.

10. Knochenschraube nach Anspruch 8 oder 9, **dadurch gekennzeichnet, daß** eine Schlitzebene im wesentlichen in einer radialen Richtung bezüglich des Schraubenkörpers (10) liegt.

11. Knochenschraube nach einem der Ansprüche 8 bis 10, **dadurch gekennzeichnet, daß** eine Schlitzebene im wesentlichen in Längsrichtung (12) bezüglich des Schraubenkörpers liegt.

12. Knochenschraube nach einem der voranstehenden Ansprüche,
**dadurch gekennzeichnet, daß** eine Mehrzahl von Längsausnehmungen (62) über den Umfang des Schraubenkörpers (10) angeordnet sind.

13. Knochenschraube nach Anspruch 12, **dadurch gekennzeichnet, daß** drei Längsausnehmungen (62) über den Umfang des Schraubenkörpers (10) angeordnet sind.

14. Knochenschraube nach Anspruch 12 oder 13, **dadurch gekennzeichnet, daß** die Mehrzahl von Längsausnehmungen (62) im wesentlichen symmetrisch zu einer Längsachse (12) des Schraubenkörpers (10) angeordnet sind.

15. Knochenschraube nach einem der voranstehenden Ansprüche,
**dadurch gekennzeichnet, daß** ein vorderer Abschnitt (24) des Schraubenkörpers (10) im wesentlichen konisch oder kegelstumpfförmig ausgebildet ist.

16. Knochenschraube nach Anspruch 15, **dadurch gekennzeichnet, daß** eine Hüllfläche (26) von Windungen (28) des Außengewindes (14) im vorderen Abschnitt (24) im wesentlichen konisch oder kegelstumpfförmig ist.

17. Knochenschraube nach Anspruch 15 oder 16, **dadurch gekennzeichnet, daß** eine Hüllfläche (30) des Schraubenkörpers (10) im vorderen Abschnitt (24) im wesentlichen konisch oder kegelstumpfförmig ist.

18. Knochenschraube nach einem der voranstehenden Ansprüche,
**dadurch gekennzeichnet, daß** eine Hüllfläche (36) von Windungen im mittleren Abschnitt (22) im wesentlichen zylindrisch ist.

19. Knochenschraube nach einem der voranstehenden Ansprüche,
**dadurch gekennzeichnet, daß** eine Hüllfläche des Schraubenkörpers (10) im mittleren Abschnitt (22) im wesentlichen zylindrisch ist.

20. Knochenschraube nach einem der voranstehenden Ansprüche,
**dadurch gekennzeichnet, daß** ein Hohlkanal (50) im Schraubenkörper (10) durch den mittleren Abschnitt (22) geführt ist.

21. Knochenschraube nach einem der voranstehenden Ansprüche,
**dadurch gekennzeichnet, daß** eine Hüllfläche (44) des Schraubenkörpers (10) im hinteren Abschnitt (20) im wesentlichen konisch oder kegelstumpfförmig ist.

22. Knochenschraube nach einem der voranstehenden Ansprüche,
**dadurch gekennzeichnet, daß** eine Hüllfläche (36) von Windungen (42) des Außengewindes (14) im hinteren Abschnitt (20) im wesentlichen zylindrisch ist.

23. Knochenschraube nach Anspruch 22, **dadurch gekennzeichnet, daß** eine Hüllfläche von Windungen (42) im hinteren Abschnitt (20) und von Windungen im mittleren Abschnitt (22) zusammenfällt.

24. Knochenschraube nach einem der voranstehenden Ansprüche,
**dadurch gekennzeichnet, daß** ein Konuswinkel des vorderen Abschnitts (24) im wesentlichen einem Konuswinkel des hinteren Abschnitts (20) entspricht.

25. Knochenschraube nach einem der voranstehenden Ansprüche,
**dadurch gekennzeichnet, daß** Windungen (16) des Außengewindes (14) in einem Bereich (24), in dem keine Längsausnehmungen (62) angeordnet sind, mit Längsdurchbrechungen (72) versehen sind.

26. Knochenschraube nach einem der voranstehenden Ansprüche,
**dadurch gekennzeichnet, daß** eine Oberfläche (32, 40, 46) des Schraubenkörpers (10) zwischen benachbarten Windungen (16) des Außengewindes (14) im wesentlichen parallel zur Längsrichtung (12) des Schraubenkörpers (10) ist.

27. Knochenschraube nach einem der Ansprüche 4 bis 26, **dadurch gekennzeichnet, daß** der Hohlkanal (50) durch eine Sacklochbohrung gebildet ist.

28. Knochenschraube nach einem der voranstehenden Ansprüche,
**dadurch gekennzeichnet, daß** der Schraubenkörper (10) mit einem Innengewinde versehen ist.

29. Knochenschraube nach einem der voranstehenden Ansprüche,
**dadurch gekennzeichnet, daß** ein Schraubenkopf (58; 60) zum Einsatz in den Schraubenkörper (10) mit einem Kanal versehen ist.

30. Knochenschraube nach einem der voranstehenden Ansprüche,
**dadurch gekennzeichnet, daß** der Schraubenkörper (10) ein Ankopplungselement (74) für einen Knochenzementapplikator (76) aufweist.

31. Knochenschraube nach Anspruch 30, **dadurch gekennzeichnet, daß** das Ankopplungselement (74) eine Aufnahme (78) für eine Düse (80) eines Knochenzementapplikators (76) umfaßt.

32. Knochenschraube nach einem der voranstehenden Ansprüche,
**dadurch gekennzeichnet, daß** ein Durchmesser des Schraubenkörpers (10) größer ist als eine Ganghöhe des Außengewindes (14).

33. Knochenschraube nach einem der voranstehenden Ansprüche,
**dadurch gekennzeichnet, daß** eine Knochenschraube in etwa sieben bis zwölf Windungsumläufe des Außengewindes (14) umfaßt.

34. Knochenschraube nach einem der voranstehenden Ansprüche,
**dadurch gekennzeichnet, daß** das Außengewinde (14) so ausgebildet ist, daß die Knochenschraube selbstschneidend ist.

## Claims

1. A bone-screw with a screw body (10) provided with an external thread (14) and which has flow-through means for bone cement for producing a cement casing around the bone-screw, **characterized in that** the bone-screw has portions (20, 22, 24) following one another in the longitudinal direction (12) of the screw body (10), which portions differ from one another in the formation of an outer surface, **in that** the flow-through means comprise at least one longitudinal recess (62) formed crosswise to a radial direction (64) and crosswise to a circumferential direction (66) in the screw body (10), **in that** the at least one longitudinal recess (62) is, in its longitudinal direction (68), dimensioned so that it extends over a plurality of turns (16) of the external thread (14), **in that** a middle portion (22) of the screw body (10) is of a substantially cylindrical design, **in that** a posterior portion (20) of the screw body (10) is substantially conical or shaped like a truncated cone, and **in that** the at least one longitudinal recess (62) is arranged substantially in the middle portion (22).

2. A bone-screw according to Claim 1, **characterized in that** the at least one longitudinal recess (62) is arranged perpendicularly to a radial direction (64).

3. A bone-screw according to Claim 1 or 2, **characterized in that** the at least one longitudinal recess (62) is arranged perpendicularly to a circumferential direction (66).

4. A bone-screw according to one of the preceding Claims, **characterized in that** the flow-through means comprise a hollow channel (50) formed within the screw body (10) and that the at least one longitudinal recess (62) is connected to the hollow channel (50).

5. A bone-screw according to Claim 4, **characterized in that** the at least one longitudinal recess (62) is connected to the hollow channel (50) in its longitudinal direction (68).

6. A bone-screw according to one of the preceding Claims, **characterized in that** corresponding recesses (70) in turns (16) of the external thread (14) are associated with the at least one longitudinal recess (62).

7. A bone-screw according to Claim 6, **characterized in that** a turn (18) of the external thread (14) is broken through in the region of the at least one longitudinal recess (62).

8. A bone-screw according to one of the preceding Claims, **characterized in that** the at least one longitudinal recess (62) is in the form of a slit.

9. A bone-screw according to Claim 8, **characterized in that** one slit plane is substantially perpendicular to a circumferential direction of the screw body (10).

10. A bone-screw according to Claim 8 or 9, **characterized in that** one slit plane lies substantially in a radial direction relative to the screw body (10).

11. A bone-screw according to one of Claims 8 to 10, **characterized in that** one slit plane lies substantially in a longitudinal direction (12) relative to the screw body.

12. A bone-screw according to one of the preceding Claims, **characterized in that** a plurality of longitudinal recesses (62) are arranged over the circumference of the screw body (10).

13. A bone-screw according to Claim 12, **characterized in that** three longitudinal recesses (62) are arranged over the circumference of the screw body (10).

14. A bone-screw according to Claim 12 or 13, **characterized in that** the majority of longitudinal recesses (62) are arranged substantially symmetrically to a longitudinal axis (12) of the screw body (10).

15. A bone-screw according to one of the preceding Claims, **characterized in that** an anterior portion (24) of the screw body (10) is substantially conical or shaped like a truncated cone.

16. A bone-screw according to Claim 15, **characterized in that** an enveloping surface (26) of turns (28) of the external thread (14) in the anterior portion (24) is substantially conical or shaped like a truncated cone.

17. A bone-screw according to Claim 15 or 16, **characterized in that** an enveloping surface (30) of the screw body (10) in the anterior portion (24) is substantially conical or shaped like a truncated cone.

18. A bone-screw according to one of the preceding Claims, **characterized in that** an enveloping surface (36) of turns in the middle portion (22) is substantially cylindrical.

19. A bone-screw according to one of the preceding Claims, **characterized in that** an enveloping surface of the screw body (10) in the middle portion (22) is substantially cylindrical.

20. A bone-screw according to one of the preceding Claims, **characterized in that** a hollow channel (50) in the screw body (10) is guided through the middle portion (22).

21. A bone-screw according to one of the preceding Claims, **characterized in that** an enveloping surface (44) of the screw body (10) in the posterior portion (20) is substantially conical or shaped like a truncated cone.

22. A bone-screw according to one of the preceding Claims, **characterized in that** an enveloping surface (36) of turns (42) of the external thread (14) in the posterior portion (20) is substantially cylindrical.

23. A bone-screw according to Claim 22, **characterized in that** an enveloping surface of turns (42) in the posterior portion (20) and of turns in the middle portion (22) coincides.

24. A bone-screw according to one of the preceding Claims, **characterized in that** a cone angle of the anterior portion (24) substantially corresponds to a cone angle of the posterior portion (20).

25. A bone-screw according to one of the preceding Claims, **characterized in that**, in a region (24) in which no longitudinal recesses (62) are arranged, turns (16) of the external thread (14) are provided with longitudinal break-throughs (72).

26. A bone-screw according to one of the preceding Claims, **characterized in that** a surface (32, 40, 46) of the screw body (10) between adjacent turns (16) of the external thread (14) is substantially parallel to the longitudinal direction (12) of the screw body (10).

27. A bone-screw according to one of Claims 4 to 26, **characterized in that** the hollow channel (50) is formed by a blind bore.

28. A bone-screw according to one of the preceding Claims, **characterized in that** the screw body (10) is provided with an internal thread.

29. A bone-screw according to one of the preceding Claims, **characterized in that** a screw head (58; 60) for the purpose of insertion into the screw body (10) is provided with a channel.

30. A bone-screw according to one of the preceding Claims, **characterized in that** the screw body (10) has a coupling member (74) for a bone-cement applicator (76).

31. A bone-screw according to Claim 30, **characterized in that** the coupling member (74) comprises a seat (78) for a nozzle (80) of a bone-cement applicator (76).

32. A bone-screw according to one of the preceding Claims, **characterized in that** a diameter of the screw body (10) is larger than a lead of the external thread (14).

33. A bone-screw according to one of the preceding Claims, **characterized in that** a bone-screw comprises roughly seven to twelve turns of the external thread (14).

34. A bone-screw according to one of the preceding Claims, **characterized in that** the external thread (14) is designed in such a way that the bone-screw is self-cutting.

## Revendications

1. Vis à os comportant un corps de vis (10) qui est pourvu d'un pas de vis extérieur (14) et qui présente des moyens d'écoulement pour du ciment à os pour réaliser une enveloppe de ciment autour de la vis à os, **caractérisée en ce que** la vis à os comprend des tronçons (20, 22, 24) se succédant en direction longitudinale (12) du corps de vis (10), qui se distinguent dans la réalisation d'une surface extérieure, **en ce que** les moyens d'écoulement comprennent au moins un évidement longitudinal (62) qui est formé dans le corps de vis (10) transversalement à une direction radiale (64) et transversalement à une direction périphérique (66), **en ce que** ledit au moins un évidement longitudinal (62) a des dimensions telles dans sa direction longitudinale (68) qu'il s'étend sur plusieurs filets (16) du pas de vis extérieur (14), **en ce qu'**un tronçon médian (22) du corps de vis (10) est réalisé sensiblement cylindrique, **en ce qu'**un tronçon postérieur (20) du corps de vis (10) est réalisé de forme sensiblement conique ou tronconique, et **en ce que** ledit au moins un évidement longitudinal (62) est agencé sensiblement dans le tronçon médian (22).

2. Vis à os selon la revendication 1, **caractérisée en ce que** ledit au moins un évidement longitudinal (62) est agencé perpendiculairement à une direction radiale (64).

3. Vis à os selon l'une ou l'autre des revendications 1 et 2, **caractérisée en ce que** ledit au moins un évidement longitudinal (62) est agencé perpendiculairement à une direction périphérique (66).

4. Vis à os selon l'une des revendications précédentes, **caractérisée en ce que** les moyens d'écoulement comprennent un canal creux (50) formé dans le corps de vis (10) et **en ce que** ledit au moins un évidement longitudinal (62) est relié au canal creux (50).

5. Vis à os selon la revendication 4, **caractérisée en ce que** ledit au moins un évidement longitudinal (62) est relié au canal creux (50) dans sa direction longitudinale (68).

6. Vis à os selon l'une des revendications précédentes, **caractérisée en ce que** des évidements (70) correspondants dans des filets (16) du pas de vis extérieur (14) sont associés audit au moins un évidement longitudinal (62).

7. Vis à os selon la revendication 6, **caractérisée en ce qu'**un filet (18) du pas de vis extérieur (14) est interrompu dans la région dudit au moins un évidement longitudinal (62).

8. Vis à os selon l'une des revendications précédentes, **caractérisée en ce que** ledit au moins un évidement longitudinal (62) est réalisé en forme de fente.

9. Vis à os selon la revendication 8, **caractérisée en ce qu'**un plan de fente est sensiblement perpendiculaire à une direction périphérique du corps de vis (10).

10. Vis à os selon l'une ou l'autre des revendications 8 et 9, **caractérisée en ce qu'**un plan de fente est situé sensiblement dans une direction radiale par rapport au corps de vis (10).

11. Vis à os selon l'une des revendications 8 à 10, **caractérisée en ce qu'**un plan de fente est situé sensiblement en direction longitudinale (12) par rapport au corps de vis (10).

12. Vis à os selon l'une des revendications précédentes, **caractérisée en ce qu'**une pluralité d'évidements longitudinaux (62) sont agencés sur la périphérie du corps de vis (10).

13. Vis à os selon la revendication 12, **caractérisée en ce que** trois évidements longitudinaux (62) sont agencés sur la périphérie du corps de vis (10).

14. Vis à os selon l'une ou l'autre des revendications 12 et 13, **caractérisée en ce que** la plupart des évidements longitudinaux (62) sont agencés sensiblement symétriques à un axe longitudinal (12) du corps de vis (10).

15. Vis à os selon l'une des revendications précédentes, **caractérisée en ce qu'**un tronçon antérieur (24) du corps de vis (10) est réalisé de forme sensiblement conique ou tronconique.

16. Vis à os selon la revendication 15, **caractérisée en ce qu'**une surface enveloppe (26) de filets (28) du pas de vis extérieur (14) dans le tronçon antérieur (24) est de forme sensiblement conique ou tronconique.

17. Vis à os selon l'une ou l'autre des revendications 15 et 16, **caractérisée en ce qu'**une surface enveloppe (30) du corps de vis (10) dans le tronçon antérieur (24) est de forme sensiblement conique ou tronconique.

18. Vis à os selon l'une des revendications précédentes, **caractérisée en ce qu'**une surface enveloppe (36) de filets dans le tronçon médian (22) est sensiblement cylindrique.

19. Vis à os selon l'une des revendications précédentes, **caractérisée en ce qu'**une surface enveloppe du corps de vis (10) dans le tronçon médian (22) est sensiblement cylindrique.

20. Vis à os selon l'une des revendications précédentes, **caractérisée en ce qu'**un canal creux (50) dans le corps de vis (10) est guidé à travers le tronçon médian (22).

21. Vis à os selon l'une des revendications précédentes, **caractérisée en ce qu'**une surface enveloppe (44) du corps de vis (10) dans le tronçon postérieur (20) est sensiblement conique ou tronconique.

22. Vis à os selon l'une des revendications précédentes, **caractérisée en ce qu'**une surface enveloppe (36) de filets (42) du pas de vis extérieur (14) dans le tronçon postérieur (20) est sensiblement cylindrique.

23. Vis à os selon la revendication 22, **caractérisée en ce qu'**une surface enveloppe de filets (42) dans le tronçon postérieur (20) et une surface enveloppe de filets dans le tronçon médian (22) coïncident.

24. Vis à os selon l'une des revendications précédentes, **caractérisée en ce qu'**un angle conique du tronçon antérieur (24) correspond sensiblement à un angle conique du tronçon postérieur (20).

25. Vis à os selon l'une des revendications précédentes, **caractérisée en ce que** des filets (16) du pas de vis extérieur (14) sont pourvus de percées longitudinales (72) dans une région (24) dans laquelle ne sont pas agencés d'évidements longitudinaux (62).

26. Vis à os selon l'une des revendications précédentes, **caractérisée en ce que** une surface (32, 40, 46) du corps de vis (10) entre des filets (16) voisins du pas de vis extérieur (14) est sensiblement parallèle à la direction longitudinale (12) du corps de vis (10).

27. Vis à os selon l'une des revendications 4 à 26, **caractérisée en ce que** le canal creux (50) est formé par un trou borgne.

28. Vis à os selon l'une des revendications précédentes, **caractérisée en ce que** le corps de vis (10) est pourvu d'un pas de vis intérieur.

29. Vis à os selon l'une des revendications précédentes, **caractérisée en ce qu'**une tête de vis (58 ; 60) est pourvue d'un canal pour être insérée dans le corps de vis (10).

30. Vis à os selon l'une des revendications précédentes, **caractérisée en ce que** le corps de vis (10) présente un élément d'accouplement (74) pour un applicateur de ciment à os (76).

31. Vis à os selon la revendication 30, **caractérisée en ce que** l'élément d'accouplement (74) comprend un logement (78) pour une tuyère (80) d'un applicateur de ciment à os (76).

32. Vis à os selon l'une des revendications précédentes, **caractérisée en ce qu'**un diamètre du corps de vis (10) est supérieur à une hauteur de pas du pas de vis extérieur (14).

33. Vis à os selon l'une des revendications précédentes, **caractérisée en ce qu'**une vis à os comprend approximativement sept à douze spires de filets du pas de vis extérieur (14).

34. Vis à os selon l'une des revendications précédentes, **caractérisée en ce que** le pas de vis extérieur (14) est réalisé de telle sorte que la vis à os est autotaraudeuse.
